(19) Europäisches Patentamt

European Patent Office

Office européen des brevets

(11) Veröffentlichungsnummer: **0 388 963 B1**

(12) **EUROPÄISCHE PATENTSCHRIFT**

(45) Veröffentlichungstag der Patentschrift: **25.05.94**

(21) Anmeldenummer: **90105447.8**

(22) Anmeldetag: **22.03.90**

(51) Int. Cl.⁵: **C12N 15/70**, C12N 15/67

(54) Verfahren zur Expression eines rekombinanten Gens.

(30) Priorität: **23.03.89 DE 3909710**

(43) Veröffentlichungstag der Anmeldung:
**26.09.90 Patentblatt 90/39**

(45) Bekanntmachung des Hinweises auf die
Patenterteilung:
**25.05.94 Patentblatt 94/21**

(84) Benannte Vertragsstaaten:
**AT BE CH DE DK ES FR GB GR IT LI LU NL SE**

(56) Entgegenhaltungen:

**NUCLEIC ACIDS RESEARCH, Band 13, Nr. 11,
1985, Seiten 4113-4123, IRL Press Ltd,Oxford,
GB; F. BONEKAMP et al.: "Codon-defined ribosomal pausing in Escherichiacoli detected by using the pyr E attennator to probe
the coupling between transcription and
translation"**

**NUCLEIC ACIDS RESEARCH, Band 12, Nr. 17,
1984, Seiten 6663-6671, IRL Press Ltd, Oxford, GB; M. ROBINSON et al.: "Codon usage
can affect efficiency of translation of genes
in Escherichia coli"**

(73) Patentinhaber: **BOEHRINGER MANNHEIM
GMBH**

**D-68298 Mannheim(DE)**

(72) Erfinder: **Brinkmann, Ulrich, Dipl.-Biologe
Von-Siemens-Strasse 5
D-4700 Hamm(DE)**
Erfinder: **Mattes, Ralf, Prof.Dr.rer.nat.
Friedrich-Zundel-Strasse 14
D-7000 Stuttgart 75(DE)**
Erfinder: **Stern, Anne, Dr.rer.nat.
Karwendelstrasse 10
D-8122 Penzberg(DE)**

(74) Vertreter: **Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B. Böhm
Postfach 86 08
20
D-81635 München (DE)**

**Beschreibung**

Die Erfindung betrifft ein Verfahren zur Expression eines rekombinanten Gens, das AGA- oder/und AGG-Kodons für Arginin enthält, in E. coli nach Transformation mit einem Expressionsvektor, der das rekombinante Gen enthält.

Die gentechnologische Herstellung von Proteinen oder proteinhaltigen Genprodukten ist eines der Hauptziele der modernen Biotechnologie. Da sich vor allem Prokaryonten aufgrund ihrer leichten Fermentierbarkeit zur Herstellung großer Proteinmengen eignen, wurde bereits vielfach versucht, auch eukaryontische Gene in Prokaryonten zu exprimieren. Hierbei ergeben sich jedoch Schwierigkeiten dahingehend, daß eukaryontische Proteine in Prokaryonten-Zellen oft nur in geringen Mengen hergestellt werden und die Zellen Fermentations/Wachstumsschwierigkeiten bei erhöhter Produktion der Proteine zeigen.

Derartige Probleme bei der Expression werden unter anderem bei dem Gewebs(tissue type)-Plasminogen-Aktivator t-PA beobachtet. Die Herstellung dieses Proteins ist jedoch ein erstrebenswertes Ziel, da es sich bei der klinischen Erprobung als zur Behandlung von Infarktkrankheiten geeignet erwiesen hat. Obwohl E. coli-Zellen die auf einem Plasmid oder Vektor eingeführte t-PA-cDNA in an sich befriedigender Menge exprimieren und t-PA produzieren können, lassen die Zellen jedoch bei der Bildung von Biomasse während der Fermentation stark nach, so daß insgesamt die Produktion von t-PA nicht den Stand erreicht, den man erwarten könnte, wenn man von der Produktion einer einzelnen Zelle ausgeht. Auch ist die Plasmidstabilität in den E. coli-Zellen gering und durch Verlust des kodierenden Plasmids nimmt die Produktionsrate weiter ab. Bisher ist es daher nur möglich, eine Expression von ca. 5 % des Gesamtzellproteins an t-PA zu erreichen (Rothstein und Bertonis, Gene 61 (1987), 41-50).

Der Erfindung liegt deshalb die Aufgabe zugrunde, ein Verfahren bereitzustellen, das es ermöglicht, rekombinante Gene, deren Expression nach den bisher bekannten Verfahren zu einem deutlich verschlechterten Wachstum der Wirtszellen und Plasmidinstabilität und damit zu geringen Ausbeuten an Genprodukt führte, mit erhöhter Ausbeute zu exprimieren.

Gelöst wird diese Aufgabe erfindungsgemäß durch ein Verfahren zur Expression eines rekombinanten Gens, das AGA- oder/und AGG-Kodons für Arginin enthält, in E. coli nach Transformation mit einem Expressionsvektor der das rekombinante Gen enthält, das dadurch gekennzeichnet ist, daß man bei einem Gehalt von mehr als 3 % an AGA- oder/und AGG-Kodons im rekombinanten Gen die Menge dieser Kodons auf höchstens 3 %, bezogen auf die Gesamtmenge der Kodons im Gen, vermindert.

Die Erfindung beruht auf der Erkenntnis, daß bei Genen, die mehr als 3 % ihrer Kodons in Form von AGA- oder/und AGG-Kodons enthalten, eine wesentlich bessere Ausbeute in E. coli erreicht werden kann, wenn man den prozentualen Anteil der AGA- oder/und AGG-Kodons auf 3 % oder weniger senkt. Versuche haben gezeigt, daß eine Verminderung der Menge dieser Kodons von z.B. 4 % auf 3 % eine Steigerung der Ausbeute auf das Achtfache, eine Verminderung auf 2 % eine Steigerung der Ausbeute auf mehr als das Zehnfache bewirken kann.

Das erfindungsgemäße Verfahren ermöglicht es, die Produkte rekombinanter Gene, deren Expression bisher aufgrund von schlechtem Wachstum der E. coli-Wirtszellen nur in geringen Ausbeuten zu den gewünschten Proteinen führte, in deutlich erhöhten Mengen herzustellen.

Das erfindungsgemäße Verfahren eignet sich für alle rekombinanten Gene, die mehr als 3 % AGA-oder/und AGG-Kodons enthalten. Vorzugsweise werden eukaryontische Gene, insbesondere deren cDNA exprimiert. Dies sind z.B. menschliche Urokinase, t-PA oder Derivate davon, HIV-Proteine (z.B. gp41, p24), alpha-Glucosidase aus Hefe oder aus E. coli. Ein großer Vorteil des erfindungsgemäßen Verfahrens liegt hierbei auch darin, daß die rekombinanten Gene konstitutiv exprimiert werden können.

Besonders bevorzugt ist das erfindungsgemäße Verfahren zur Herstellung von t-PA, indem man als rekombinantes Gen das t-PA-Gen (beschrieben von Pennica et al., Nature 301 (1983) 214-221) oder eine Variante davon exprimiert. Gegenüber der bisher im Stand der Technik bekannten Expressionsrate von etwa 5 % t-PA pro E. coli Gesamtzellprotein kann erfindungsgemäß die Ausbeute auf mehr als 30 % gesteigert werden.

Die Verminderung des Gehalts an AGG- und/oder AGA-Kodons kann hierbei auf verschiedene Weise durchgeführt werden. In einer bevorzugten Ausführungsform der Erfindung werden zur Verminderung der Menge überzählige Kodons, d.h. so viele Kodons, bis die Menge der verbleibenden AGG- oder/und AGA-Kodons höchstens 3 % beträgt, gegen andere für Arginin kodierende Kodons ausgetauscht. Die weiteren für Arginin kodierenden Tripletts sind CGA, CGC, CGG und CGT. Hierbei ist ein Austausch gegen das CGT-Kodon besonders bevorzugt.

In einer anderen bevorzugten Ausführungsform der Erfindung werden zur Verminderung der AGA-oder/und AGG-Kodonmenge überzählige Kodons gegen Kodons für andere Aminosäuren ausgetauscht, welche die Aktivität des Proteins nicht oder nur unwesentlich beeinträchtigen. Für einen Großteil der heutzutage in rekombinanter Form exprimierbaren Gene und den daraus erhaltenen Proteinen ist nämlich bereits bekannt, welche Proteinregionen für die Aktivität unbedingt erforderlich sind, und welche Regionen eventuell verändert werden können. So ist beispielsweise für t-PA bekannt, daß Mutationen durchgeführt werden können, ohne die Aktivität des Proteins zu beeinträchtigen (vgl. Fibrinolysis 2 (1988) 133-142 und die darin zitierten Literaturstellen).

Für manche Proteine (vgl. z.B. EP-A 0 291 055, DE 38 33 601) ist darüberhinaus bekannt, daß sie durch den Austausch einer oder mehrerer Aminosäuren sogar in ihrer Aktivität verbessert werden. Es ist daher erfindungsgemäß bevorzugt, daß man die AGA- oder/und AGG-Kodons gegen Kodons für andere Aminosäuren austauscht, welche die Aktivität des exprimierten Proteins positiv beeinflussen.

In wiederum einer anderen bevorzugten Ausführungsform der Erfindung wird die Verminderung der AGG- und/oder AGA-Kodons dadurch erreicht, daß die überzähligen Kodons gegen Stop-Kodons ausgetauscht werden und man dann E. coli- Stämme als Wirtszellen verwendet, welche diese Stop-Kodons supprimieren. Beispiele hierfür sind die E. coli-Stämme C600, HB101, DH2, JM109, JM 103 (supE); SC122,K165 (supC), welche die Stop-Kodons UAA/UAG (supC) und UAG (supE) supprimieren (B. Bachmann, Seiten 807 bis 876 und Seiten 1190 bis 1219; S.R. Kushner, Seiten 1225 bis 1230 in Escherichia Coli and Salmonella Typhimurium, Cellular and Molecular Biology, Band 2, Editor F.C. Neidhardt, American Society for Microbiology 1987).

In einer weiteren besonders bevorzugten Ausführungsform der Erfindung werden zur Verminderung der AGA- oder/und AGG-Kodons überschüssige Kodons deletiert. Eine derartige Deletion kann entsprechend den zum Austausch gegen Kodons, die für andere Aminosäuren kodieren, relevanten Kriterien durchgeführt werden. Da jedoch möglicherweise durch die Deletion sehr vieler einzelner Kodons sich Auswirkungen auf die Tertiärstruktur des exprimierten Proteins ergeben können, kann hier gegebenenfalls die Deletion mit dem Austausch gegen andere für Arginin kodierende Kodons oder gegen für andere Aminosäuren kodierende Kodons kombiniert werden.

Sowohl der Austausch der AGG- oder/und AGA-Kodons gegen andere Kodons als auch die Deletion dieser Kodons wird erfindungsgemäß bevorzugt über gerichtete Mutagenese (site directed mutagenesis, siehe Morinaga et al., Biotechnol. 2 (1984), 6, 634-639) bewirkt. Die Überprüfung der Aktivität der erhaltenen Proteine erfolgt dann nach an sich bekannten Methoden.

Eine weitere Möglichkeit zur Verminderung des Gehalts an AGG- und/oder AGA-Kodons und damit eine weitere bevorzugte Ausführungsform der Erfindung liegt in einem Verfahren, bei dem man die Menge der im rekombinanten Gen enthaltenen AGA- oder/und AGG-Kodons dadurch vermindert, daß man eine für das gewünschte Protein kodierende DNA-Sequenz unter Berücksichtigung der Vermeidung eines Gehaltes an AGG- oder/und AGA-Kodons von mehr als 3 %, insbesondere von mehr als 2 %, chemisch synthetisiert. Eine derartige chemische Synthese erscheint vor allem bei relativ kurzen Genen vielversprechend und vom Arbeitsaufwand möglicherweise am geringsten belastend. Anstelle der AGA/AGG-Kodons wird dann gleich bei der Synthese entsprechend den zum Austausch der Kodons genannten Kriterien vorgegangen.

Durch das erfindungsgemäße Verfahren zur Expression von rekombinanten Genen, gelingt es also, auch solche Gene effektiv in die entsprechenden Proteine überzuführen, welche in ihrer natürlichen Form mehr als 3 % AGA-oder/und AGG-Kodons enthalten. Vor allem bei dem Protein t-PA, welches ein aussichtsreicher Wirkstoff in Arzneimitteln zur Thrombolyse ist, sind relativ viele derartige Kodons enthalten, und es gelingt durch Verminderung dieser Kodons in der erfindungsgemäßen Weise, eine außerordentlich starke Erhöhung der Expressionsrate dieses Proteins zu bewirken.

Die folgenden Beispiele in Verbindung mit den Abbildungen erläutern die Erfindung weiter:

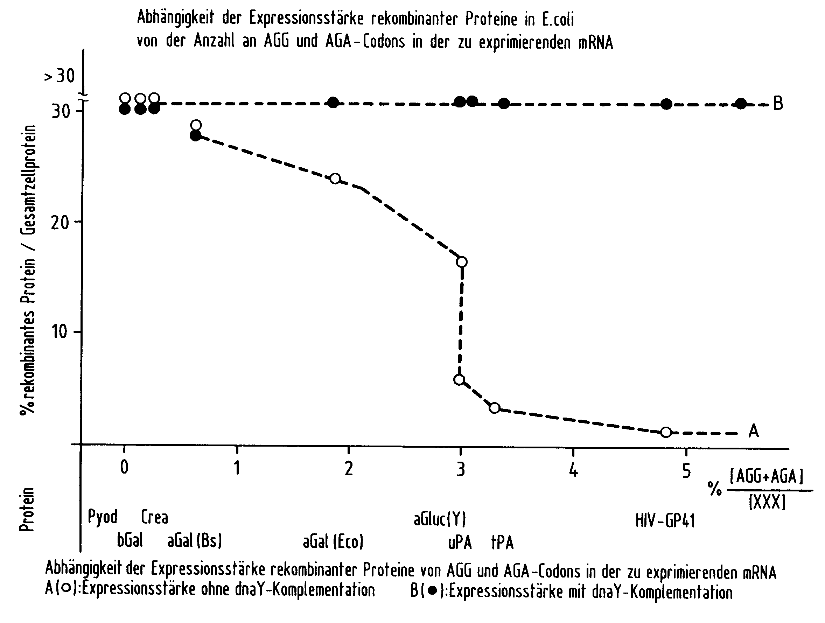

Fig. 1     zeigt den Vergleich der Expressionsausbeute verschiedener Gene in Bezug auf ihren Gehalt an AGG/AGA-Kodons.

**Beispiel 1**

Vergleich der Menge an AGG/AGA-Kodons in zu exprimierenden Genen und der jeweils erreichten Expressionsausbeuten

Die Plasmide pUPA 110 (enthaltend die Sequenz für menschliche Pro-Urokinase ohne Signalpeptid, Holmes et al., Bio/Technology 3 (1985) 923-929), pGP 41 (enthaltend die kodierende Sequenz für das GP-41-Protein des menschlichen HIV-Virus, Ratner et al., Nature 313 (1985) 277-284)), pKK 177-3/GLUCPI (enthaltend die Sequenz für alpha-Glucosidase aus Hefe, EP-A 0 300 425); pBT102 (DSM 2091) (alpha-Galactosidase aus E. coli) und Plasmid p UR 289 (Rüther und Müller-Hill, EMBO J. 2 (1983) 1791-1794) (enthaltend die Sequenz für beta-Galactosidase aus E. coli) wurden in E. coli, DSM 3689, transformiert, der ein Iq-Plasmid enthält. Transformanten wurden auf LB-Platten (Manniatis et al., (1982) Molecular Cloning: A Laboratory Manual, Cold Spring Harbor Laboratory, Cold Spring Harbor) mit 25 $\mu$g/ml Kanamycin bzw. 50 $\mu$g/ml Ampicillin angezogen. Plasmidhaltige Zellen wurden in LB bis zur $OD_{550}$ nm = 0,3 angezogen, induziert (Zusatz von 10 mmol/l IPTG) und bei 37°C fermentiert. Das Wachstum der Kulturen wurde durch Kontrolle der Zelldichte ($OD_{550}$) in regelmäßigen Abständen überwacht.

Vier Stunden nach Induktion wurden die Zellen geerntet und durch Ultraschallbehandlung aufgeschlossen. Das so gewonnene Zell-Lysat wurde elektrophoretisch analysiert (Coomassie-Blau gefärbte SDS-Gele), die Proteine wurden in Westernblots mit polyklonalen Antikörpern aus Ziege nachgewiesen und über densitometrische Auswertung coomassiegefärbter SDS-Gele nach vorhergegangener Proteinbestimmung des Zell-Lysates quantifiziert. Der Vergleich zwischen AGG/AGA-Kodonmenge in der mRNA und der jeweils erreichten Expressionsausbeute ist in Fig. 1 dargestellt. Hieraus wird offensichtlich, daß eine eindeutige reverse Korrelation besteht zwischen der relativen (prozentualen) Menge an AGG/AGA-Kodons in zu exprimierender rekombinanter mRNA und der Expressionsausbeute dieser. Steigt die Menge der AGG/AGA-Argininkodons auf größer 2 %, bezogen auf die gesamte Anzahl der Kodons an, so bewirkt dieses eine deutliche Reduzierung der Expressionsleistung.

**Beispiel 2**

Anhand der im Beispiel 1 gezeigten Daten wird deutlich, daß die Menge der AGG- + AGA-Kondons erst ab 2 % des rekombinanten Gens expressionslimitierend wirkt, darunter jedoch von untergeordneter Bedeutung ist. Es wurde daher ein t-PA-Expressionsplasmid hergestellt, in dem die AGG- und AGA-Argininkodons gegen andere Argininkodons ausgetauscht wurden. Hierzu wurden die Argininkodons aus dem Ausgangsplasmid pePa 126.1 (DE 38 38 378) an den in Tabelle 1 gezeigten Positionen der t-PA cDNA mit Hilfe der ebenfalls in der Tabelle beschriebenen synthetischen Deoxyoligonukleotide sukzessive nach der Methode von Morinaga et al., Biotechnol. 2 (1984), 6, 634-639 durch gerichtete Mutagenese (site directed mutagenesis) mutiert:

## T a b e l l e   1

| t-PA cDNA Position | Kodon vor Mutagenese | Kodon nach Mutagenese | Mutageneseoligonukleotid 5'..................3' |
|---|---|---|---|
| 220 | AGA | CGT | GTGATCTGCCGTGATGAAAAA |
| 270 | AGA | CGT | CCTGTGCTCCGTAGCAACCGG |
| 309 | AGG | CGT | AACAGTGGCCGTGCACAGTGC |
| 354 | AGG | CGT | AGCGAGCCACGTTGTTTCAAC |
| 455 | AGG | CGT | ATAGATACCCGTGCCACGTGC |
| 490 | AGG | CGT | ATCAGCTACCGTGGCACGTGG |
| 579/594 | AGG/AGG | CGT/CGT | CAGCGGGCGGCGTCCAGACGCCATCCGTCTGGGCCTGG |
| 624 | AGA | CGT | AACTACTGCCGTAACCCAGAT |
| 939 | AGG | CGT | AAGAACCGCCGTCTGACGTGG |
| 990 | AGA | CGT | TGCGGCCTGCGTCAGTACAGC |
| 1081-1086 | AGGAGG | CGTCGT | TGCCAAGCACCGTCGTTCGCCCGGAG |
| 1207 | AGA | CGT | ATCTTGGGCCGTACATACCGG |
| 1490 | AGA | CGT | GCTCATGTCCGTCTGTACCCA |
| 1535 | AGA | CGT | TTACTTAACCGTACAGTCACC |

EP 0 388 963 B1

Das entstehende Plasmid wurde pUBS 126.m1 benannt.

Unter denselben Expressionsbedingungen wie im Beispiel 1 beschrieben konnte mit dem Plasmid pUBS 126.m1 eine deutliche Expressionssteigerung auf ca. 30 % des Gesamtzellproteins und Vitalitätsverbesserung bei der t-PA-Produktion gegenüber der Expression mit dem Ausgangsplasmid pePa 126.1 (ca. 5 % des Gesamtzellproteins bei reduziertem Wachstum) erreicht werden.

**Patentansprüche**

1. Verfahren zur Expression eines rekombinanten Gens, das AGA- oder/und AGG-Kodons für Arginin enthält, in E. coli nach Transformation mit einem Expressionsvektor, der das rekombinante Gen enthält, **dadurch gekennzeichnet,** daß man bei einem Gehalt von mehr als 3 % an AGA- oder/und AGG-Kodons im rekombinanten Gen die Menge dieser Kodons auf höchstens 3 %, bezogen auf die Gesamtmenge der Kodons im Gen, vermindert.

2. Verfahren nach Anspruch 1, **dadurch gekennzeichnet,** daß man die AGA- oder/und AGG-Kodonmenge auf höchstens 2 % vermindert.

3. Verfahren nach Anspruch 1 oder 2, **dadurch gekennzeichnet,** daß man als rekombinantes Gen ein eukaryontisches Gen bzw. seine cDNA verwendet.

4. Verfahren nach einem der Ansprüche 1 bis 3, **dadurch gekennzeichnet,** daß man zur Verminderung der Menge überzählige AGA- oder/und AGG-Kodons gegen andere für Arginin kodierende Kodons austauscht.

5. Verfahren nach Anspruch 4, **dadurch gekennzeichnet,** daß man sie gegen CGT-Kodons austauscht.

6. Verfahren nach einem der Ansprüche 1 bis 3, **dadurch gekennzeichnet,** daß man zur Verminderung der Menge überzählige AGA- oder/und AGG-Kodons gegen Kodons für andere Aminosäuren austauscht, welche die Aktivität des Proteins nicht oder nur unwesentlich beeinträchtigen.

7. Verfahren nach einem der Ansprüche 1 bis 3, **dadurch gekennzeichnet,** daß man zur Verminderung der Menge überzählige AGG- oder/und AGA-Kodons gegen Stopkodons austauscht und zur Expression E. coli-Stämme verwendet, welche diese Stopkodons supprimieren.

8. Verfahren nach einem der Ansprüche 1 bis 3, **dadurch gekennzeichnet,** daß man die Verminderung der Menge überzähliger AGA- oder/und AGG-Kodons durch Deletion dieser Kodons, gegebenenfalls in Kombination mit einem Austausch gegen ein anderes Kodon bewirkt.

9. Verfahren nach einem der Ansprüche 4 bis 8 , **dadurch gekennzeichnet,** daß man den Austausch der AGG- oder/und AGA-Kodons gegen andere Kodons bzw. die Deletion dieser Kodons über gerichtete Mutagenese bewirkt.

10. Verfahren nach einem der Ansprüche 1 bis 3, **dadurch gekennzeichnet,** daß man ein rekombinantes Gen unter Berücksichtigung der Vermeidung von AGG- oder/und AGA-Kodons in einer Menge von mehr als 3 %, insbesondere mehr als 2 %, chemisch synthetisiert und zur Expression verwendet.

5

**Claims**

1.  Process for the expression of a recombinant gene, which contains AGA or/and AGG codons for arginine, in E. coli after transformation with an expression vector which contains the recombinant gene, **wherein** if the content of the AGA or/and AGG codons is more than 3 % in the recombinant gene, the amount of these codons is reduced to not more than 3 % relative to the total amount of the codons in the gene.

2.  Process as claimed in claim 1, **wherein** the amount of the AGA or/and AGG codons is reduced to not more than 2 %.

3.  Process as claimed in claim 1 or 2, **wherein** a eukaryotic gene or its cDNA is used as the recombinant gene.

4.  Process as claimed in one of the claims 1 to 3, **wherein** in order to reduce the amount, excess AGA or/and AGG codons are replaced by other codons coding for arginine.

5.  Process as claimed in claim 4, **wherein** they are replaced by CGT codons.

6.  Process as claimed in one of the claims 1 to 3, **wherein** in order to reduce the amount, excess AGA or/and AGG codons are replaced by codons for other amino acids which either do not impair the activity of the protein or only to a negligible extent.

7.  Process as claimed in one of the claims 1 to 3, **wherein** in order to reduce the amount, excess AGG or/and AGA codons are replaced by stop codons and E. coli strains are used for expression which suppress these stop codons.

8.  Process as claimed in one of the claims 1 to 3, **wherein** the reduction in the amount of excess AGA or/and AGG codons is achieved by deletion of these codons, if desired, in combination with a replacement by another codon.

9.  Process as claimed in one of the claims 4 to 8, **wherein** the replacement of the AGG or/and AGA codons by other codons or the deletion of these codons is achieved by site-directed mutagenesis.

10.  Process as claimed in one of the claims 1 to 3, **wherein** a recombinant gene is synthesized chemically, taking into account the avoidance of AGG or/and AGA codons in an amount of more than 3 % in particular of more than 2 %, and used for expression.

**Revendications**

1.  Procédé d'expression dans E. coli d'un gène recombinant qui contient des codons AGA et/ou AGG pour l'arginine, après transformation avec un vecteur d'expression qui contient le gène recombinant, caractérisé en ce que, pour une teneur supérieure à 3% en codons AGA et/ou AGG dans le gène recombinant, on réduit la quantité de ces codons à 3% au plus par rapport à la quantité totale des codons dans le gène.

**2.** Procédé selon la revendication 1, caractérisé en ce que l'on réduit la quantité de codons AGA et/ou AGG à 2% au plus.

**3.** Procédé selon la revendication 1 ou 2, caractérisé en ce que l'on utilise comme gène recombinant un gène eucaryotique ou son ADNc.

**4.** Procédé selon l'une des revendications 1 à 3, caractérisé en ce que, pour réduire la quantité, on remplace les codons AGA et/ou AGG en surnombre par d'autres codons codant l'arginine.

**5.** Procédé selon la revendication 4, caractérisé en ce qu'on les remplace par des codons CGT.

**6.** Procédé selon l'une des revendications 1 à 3, caractérisé en ce que, pour réduire la quantité, on remplace les codons AGA et/ou AGG en surnombre par des codons d'autres acides aminés qui ne perturbent pas l'activité de la protéine ou qui ne la perturbent que de manière insignifiante.

**7.** Procédé selon l'une des revendications 1 à 3, caractérisé en ce que, pour réduire la quantité, on remplace les codons AGG et/ou AGA en surnombre par des codons d'arrêt et on utilise pour l'expression des souches de E. coli qui suppriment ces codons d'arrêt.

**8.** Procédé selon l'une des revendications 1 à 3, caractérisé en ce que l'on réalise la réduction de la quantité des codons AGA et/ou AGG en surnombre par délétion de ces codons, éventuellement en combinaison avec un remplacement par un autre codon.

**9.** Procédé selon l'une des revendications 4 à 8, caractérisé en ce que l'on réalise le remplacement des codons AGG et/ou AGA par d'autres codons ou la délétion de ces codons par mutagenèse dirigée.

**10.** Procédé selon l'une des revendications 1 à 3, caractérisé en ce que l'on synthétise chimiquement et utilise pour l'expression un gène recombinant en tenant compte de la réduction des codons AGG et/ou AGA présents en une quantité supérieure à 3%, en particulier supérieure à 2%.

Abhängigkeit der Expressionsstärke rekombinanter Proteine in E.coli
von der Anzahl an AGG und AGA-Codons in der zu exprimierenden mRNA

Abhängigkeit der Expressionsstärke rekombinanter Proteine von AGG und AGA-Codons in der zu exprimierenden mRNA
A(o):Expressionsstärke ohne dnaY-Komplementation     B(●):Expressionsstärke mit dnaY-Komplementation